# EUROPEAN PATENT APPLICATION

(11) **EP 4 796 552 A1**
(43) Date of publication of application: **26.08.2026**
(21) Application number: 26160025.8
(22) Date of filing: 23.02.2026
(51) Int. Cl.: C07F 15/06, A61P 31/04, A61K 31/198, A61P 31/12

(54) **AN AMINO ACID-COBALT COMPLEX AND ITS USE THEREOF**

(30) Priority: 24.02.2025 US 202563762106 P; 13.02.2026 TW 115106092
(71) Applicant: Aminomatrix Labs Co., Ltd., 221425 New Taipei City Taiwan (R.O.C.) (TW)
(72) Inventor: LI, I-CHEN, 221425 New Taipei City (TW); CHEN, CHI-JUNG, 221425 New Taipei City (TW)
(74) Representative: Rapisardi, Mariacristina

(57) **Abstract**

The present disclosure provides an amino acid-cobalt complex, where the amino acid is glutamic acid or aspartic acid. The present disclosure further provides a use of the aforementioned amino acid-cobalt complex for preparing antibacterial or antiviral compositions; the use of the amino acid-cobalt complex of the present disclosure or the composition prepared therefrom can effectively inhibit the activity of various pathogenic bacteria or viruses. Furthermore, based on the characteristics of the single amino acid and its chelated metal ion, it enhances safety during individual use while providing effective antibacterial or antiviral activity.

## Description

### BACKGROUND OF THE INVENTION

### 1. Field of the Invention

The present disclosure relates to an amino acid-cobalt complex, and in particular to an amino acid-cobalt complex for preparing an antibacterial or antiviral composition.

### 2. Description of the Related Art

In recent years, people have been using a variety of drugs to treat the diseases caused by emerging pathogens; however, this has also led to the emergence of some drug-resistant or drug-tolerant pathogens, gradually reducing the effectiveness of existing medical treatments. Prior art includes studies on metal-ion-binding peptides that inhibit bacterial or viral activity through strong interactions between the peptides and cell membranes.

However, although the binding of peptides to metal ions can produce antibacterial and antiviral effects, peptides possess the ability to disrupt biological membranes, and the free radicals generated by metal ions can cause oxidative damage. Consequently, while achieving antibacterial and antiviral effects, they may also cause damage to non-target living cells. Furthermore, in terms of stability, peptides are susceptible to degradation by proteases, temperature, or pH changes. When the peptide structure is disrupted, the sites responsible for metal coordination may disappear, leading to a significant decline in antibacterial activity. This means that while the peptide may originally have been effective against a certain range of pathogens or viruses, its efficacy may be reduced to affecting only a few pathogens or viruses, or it may become completely ineffective.

Numerous studies have now demonstrated that G4 structures are present in the genomes of various viruses or in regions associated with viral replication and transcription, and are also found in nucleic acid regulatory sequences of certain bacteria or fungi; therefore, it is particularly important to develop a composition that is low in harm or low in toxicity to living organisms and that consistently inhibits a wide range of pathogenic bacteria and viruses.

### BRIEF SUMMARY OF THE INVENTION

Given the limitations and shortcomings of the prior art described above, the present disclosure provides an amino acid-cobalt complex, where the amino acid is glutamic acid or aspartic acid. Through the G-quadruplex (G4) ligand properties of the amino acid-cobalt complex, it can bind to G4 structures in various pathogens or viruses. By stabilizing or interfering with their dynamic formation, it influences their life cycles (such as replication, transcription, or gene expression), thereby achieving a broad-spectrum inhibitory effect.

In the embodiments of the present disclosure, if the amino acid is glutamic acid, its structure is represented by the following formula (I); if the amino acid is aspartic acid, its structure is represented by the following formula (II):

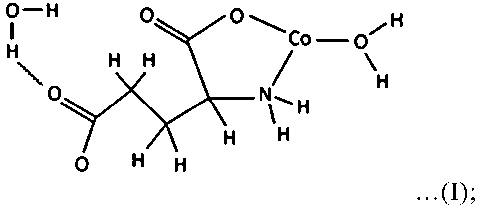

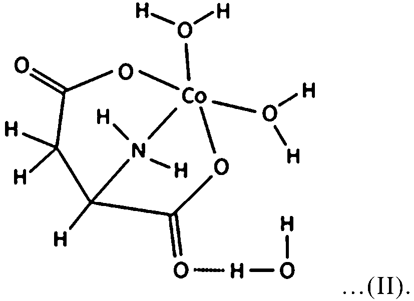

The present disclosure further provides a use of the amino acid-cobalt complex described above; where the use is for the preparation of an antibacterial or antiviral composition against bacteria or viruses.

In the embodiments of the present disclosure, the bacteria is selected from the group consisting of *Candidatus Liberibacter* spp., *Xylella fastidiosa*, *Ralstonia solanacearum*, *Clavibacter sepedonicus* (synonyms: *Clavibacter michiganensis* subsp. *sepedonicus*), *Xanthomonas* spp., *Pectobacterium carotovorum*, *Dickeya* spp., *Erwinia amylovora*, *Pseudomonas syringae*, *Puccinia* spp., *Fusarium* spp., *Botrytis cinerea*, *Phytophthora infestans*, *Magnaporthe oryzae*, *Blumeria graminis*, *Erysiphe necator*, *Alternaria* spp., *Verticillium dahliae*, *Salmonella* spp., *Pasteurella multocida*, *Bordetella bronchiseptica*, *Clostridium perfringens*, *Staphylococcus aureus*/*Staphylococcus pseudintermedius*, *Mycobacterium bovis*, *Mycobacterium avium* subspecies *paratuberculosis*, *Brucella* spp., *Enterococcus faecium, Klebsiella pneumoniae*, *Acinetobacter baumannii*, *Pseudomonas aeruginosa*, *Klebsiella aerogenes* (synonyms: *Enterobacter aerogenes*), *Escherichia coli*, *Enterococcus faecalis*, *Haemophilus influenzae*, *Candida auris*, *Candida albicans*, *Cryptococcus neoformans*, *Mycobacterium tuberculosis*, *Aspergillus fumigatus*, and *Neisseria gonorrhoeae.*

In the embodiments of the present disclosure, the virus is selected from the group consisting of avian influenza virus (AIV), foot-and-mouth disease virus (FMDV), porcine reproductive and respiratory syndrome virus (PRRSV), Newcastle disease virus (NDV), canine distemper virus (CDV), African swine fever virus (ASFV), influenza A/B/C/D virus, SARS-CoV-2, herpes simplex virus (HSV), human papilloma virus (HPV), respiratory syncytial virus (RSV), human immunodeficiency virus (HIV), hepatitis B virus (HBV), hepatitis C virus (HCV), dengue virus, and Zika virus.

In the embodiments of the present disclosure, the influenza A/B/C/D virus is H1N1 influenza A virus, H3N2 influenza A virus, or a combination thereof.

In the embodiments of the present disclosure, an antiviral concentration of the amino acid-cobalt complex is 6 to 30 mM.

In the embodiments of the present disclosure, an antibacterial concentration of the amino acid-cobalt complex is 100 mM or less.

In summary, the present disclosure provides an amino acid-cobalt complex, specifically cobalt glutamate or cobalt aspartate, which exhibits greater stability and safety compared to conventionally used peptides or combinations of peptides and metals. The present disclosure further provides a use of the amino acid-cobalt complex for preparing antibacterial or antiviral compositions. By administering a composition containing the amino acid-cobalt complex to humans, animals, or plants, significant antibacterial or antiviral activity can be achieved even at low concentrations, while reducing toxicity to the treated subjects and the possibility of developing multidrug-resistant pathogens.

### BRIEF DESCRIPTION OF THE DRAWINGS

FIG. 1 shows the results of the antiviral activity of cobalt glutamate against H1N1 of the present disclosure.
FIG. 2 shows the results of the antiviral activity of cobalt glutamate against H3N2 of the present disclosure.

### DETAILED DESCRIPTION OF THE INVENTION

It should be understood that both the general description above and the detailed description below are provided by way of example and illustration only, and are not intended to limit the scope of the claims of the present disclosure.

Unless otherwise defined, all technical and scientific terms used herein have the meanings commonly understood by those skilled in the art to which the present disclosure pertains.

The articles "a," "an," and "any" used in the present specification refer grammatically to one or more (e.g., at least one).

The primary objective of the present disclosure is to provide an amino acid-cobalt complex, where the amino acid is glutamic acid or aspartic acid.

In the embodiments of the present disclosure, the cobalt forms a complex with glutamic acid in the form of a metal ion, as shown in the following formula (I); in another embodiment of the present disclosure, the cobalt ion forms a complex with aspartic acid, as shown in the following formula (II):

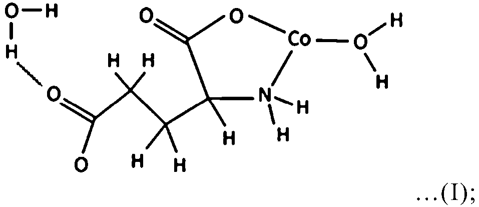

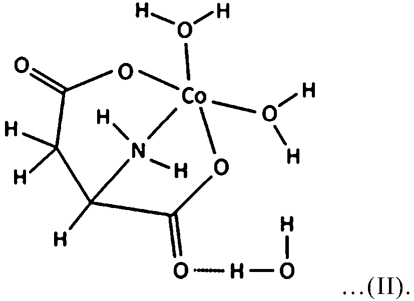

In the present specification, the term "complex" refers to a complex formed by two or more chemical substances through coordinate bonds (coordinate covalent bonds), Coulombic electrostatic interactions (e.g., ion-ion, ion-dipole, etc.), hydrogen bonds, van der Waals forces, hydrophobic interactions, or other intermolecular interactions, including but not limited to chelates.

In the present specification, the term "chelates" refers to a compound formed when a ligand binds to the same metal ion through two or more binding sites simultaneously. Specifically, the metal ion, cobalt, in the present disclosure forms a complex through chelation with the amino acids, glutamic acid or aspartic acid. This complex is more stable than the metal ion in its free state, reduces nonspecific chelation, and minimizes the oxidative stress that may be induced.

Specifically, the glutamic acid or aspartic acid used in the present disclosure is a single amino acid molecule, not a peptide. In prior art, peptides used in conventional antibacterial compositions typically achieve antibacterial and antiviral effects by binding to or inserting into cell membranes to disrupt bacterial cell membranes or viral envelope proteins and capsids; however, this also means that peptides may cause damage to non-target host cells.

The present disclosure utilizes a single amino acid, which generally enters cells via membrane transport proteins and can carry the chelated metal ions into pathogens (including bacteria, fungi, and other pathogenic microorganisms, as well as viruses) or host cells, by interacting with the pathogen's G4 structure to interfere with its replication, transcription, or gene expression, or by using metal ions to disrupt the target pathogen's metabolism, thereby achieving an inhibitory effect; or, upon entering the host cell, by altering the environment suitable for the pathogen's proliferation or activity, making it difficult for the pathogen to infect the host, thereby achieving an inhibitory effect. Therefore, compared to the peptides used in prior art, the single amino acid of the present disclosure can effectively reduce damage to the cell membranes of non-target cells (i.e., host cells), thereby achieving antibacterial or antiviral effects under conditions that are safer for the individual.

In the present specification, the term "peptide" refers to a molecule formed by two or more amino acids linked by peptide bonds.

In the present specification, the term "subject" refers to the entity to which the amino acid-cobalt complex is administered, including but not limited to humans, animals, or plants.

The present disclosure further provides a use of the amino acid-cobalt complex described above; where the use is for the preparation of antibacterial or antiviral compositions.

Specifically, this composition can be used in agriculture, poultry and livestock farming, the pet industry, or clinical medicine.

The methods of application for this composition include, but are not limited to: pesticides, irrigation solutions, foliar fertilizers, granular fertilizers, soil conditioners, concentrated suspensions, powders, and sprays in agriculture; in the farming industry as feed additives; or in clinical medicine as tablets, capsules, granules, solutions, suspensions, oral powders, intravenous injections, intramuscular injections, subcutaneous injections, intradermal injections, patches, creams, ointments, gels, sprays, sublingual tablets, nasal sprays, and suppositories.

Furthermore, when the antibacterial or antiviral compositions prepared from the amino acid-metal complexes of the present disclosure are formulated as drugs or other therapeutic formulations, they may further include a pharmaceutically acceptable excipient, which may include, but is not limited to: water, alcohols, glycols, preserving agents, antioxidants, solvent, emulsifier, suspending agent, decomposer, binding agent, excipient, stabilizing agent, chelating agent, diluent, gelling agent, preservative, lubricant, absorption enhancers, active agents, humectants, odor absorbers, fragrances, pH adjusting agents, occlusive agents, emollients, thickeners, solubilizing agents, penetration enhancers, anti-irritants, colorants, propellants, surfactant, and other similar or suitable carriers for the present disclosure.

Specifically, the present disclosure demonstrates the expected effects of cobalt glutamate and cobalt aspartate as antibacterial or antiviral compositions through the SGS antibacterial test and the plaque assay described in the following examples.

Specifically, the bacteria is selected from the group consisting of *Candidatus Liberibacter* spp., *Xylella fastidiosa*, *Ralstonia solanacearum*, *Clavibacter sepedonicus* (synonyms: *Clavibacter michiganensis* subsp. *sepedonicus*), *Xanthomonas* spp., *Pectobacterium carotovorum*, *Dickeya* spp., *Erwinia amylovora*, *Pseudomonas syringae*, *Puccinia* spp., *Fusarium* spp., *Botrytis cinerea*, *Phytophthora infestans*, *Magnaporthe oryzae*, *Blumeria graminis*, *Erysiphe necator*, *Alternaria* spp., *Verticillium dahliae*, *Salmonella* spp., *Pasteurella multocida*, *Bordetella bronchiseptica*, *Clostridium perfringens, Staphylococcus aureus*/*Staphylococcus pseudintermedius*, *Mycobacterium bovis*, *Mycobacterium avium* subspecies *paratuberculosis*, *Brucella* spp., *Enterococcus faecium*, *Klebsiella pneumoniae, Acinetobacter baumannii, Pseudomonas aeruginosa, Klebsiella aerogenes* (synonyms: *Enterobacter aerogenes*), *Escherichia coli*, *Enterococcus faecalis*, *Haemophilus influenzae*, *Candida auris*, *Candida albicans*, *Cryptococcus neoformans*, *Mycobacterium tuberculosis*, *Aspergillus fumigatus*, and *Neisseria gonorrhoeae.*

Specifically, the virus is selected from the group consisting of avian influenza virus (AIV), foot-and-mouth disease virus (FMDV), porcine reproductive and respiratory syndrome virus (PRRSV), Newcastle disease virus (NDV), canine distemper virus (CDV), African swine fever virus (ASFV), influenza A/B/C/D virus, SARS-CoV-2, herpes simplex virus (HSV), human papilloma virus (HPV), respiratory syncytial virus (RSV), human immunodeficiency virus (HIV), hepatitis B virus (HBV), hepatitis C virus (HCV), dengue virus, and Zika virus.

The influenza A/B/C/D virus includes, but is not limited to, H1N1 influenza A virus, H3N2 influenza A virus.

In the present disclosure, the aforementioned influenza viruses may include influenza A viruses, influenza B viruses, influenza C viruses, and/or influenza D viruses, as well as any of their variants, strains, subtypes, lineages, genotypes, or clades. In particular, the influenza A virus includes, but is not limited to, the H1N1 subtype, the H3N2 subtype, and their variants, or any combination thereof; the influenza B virus includes, but is not limited to, the Victoria lineage and/or the Yamagata lineage, or any combination thereof; and the influenza C virus and the influenza D virus may be any genotype, lineage, or clade, or any combination thereof.

The amino acid-cobalt complexes may be applied at concentrations of 0.01 mM, 0.012 mM, 0.025 mM, 0.050 mM, 0.075 mM, 0.1 mM, 0.12 mM, 0.25 mM, 0.50 mM, 0.75 mM, 1 mM, 1.2 mM, 2.5 mM, 6.0 mM, 7.5 mM, 10 mM, 15 mM, 20 mM, 25 mM, 30 mM, 35 mM, 40 mM, 45 mM, 50 mM, 55 mM, 60 mM, 65 mM, 70 mM, 75 mM, 80 mM, 85 mM, 90 mM, 95 mM, 100 mM, 120 mM, or any value within the range of 0.01-120 mM, or an interval comprising any two such values; in the embodiments of the present disclosure, the preferred concentration for antiviral activity is 6 to 30 mM, and the preferred concentration for antibacterial activity is 100 mM or less.

Specifically, administering the amino acid-metal complex of the present disclosure to an individual via the aforementioned methods allows for the achievement of antibacterial or antiviral effects under conditions that are more stable and safer than those of prior art.

### Examples

In the embodiments of the present disclosure, the aforementioned glutamic acid or aspartic acid and a source of cobalt metal, such as an oxide, hydroxide, or pure metal, are used based on the molecular weight of glutamic acid (147.13 g/mol), the molecular weight of aspartic acid (133.1 g/mol), and the molecular weight of the cobalt ion Co²⁺ (58.93 g/mol) to calculate the required molar amounts, and the chelation is carried out using methods well known to those skilled in the art to obtain a 0.1 M solution of cobalt glutamate and cobalt aspartate.

### Example 1. SGS Antibacterial Test Results for Cobalt Glutamate and Cobalt Aspartate

### 1-1. Preparation of the inoculum

In the present embodiment, cultured *Candida albicans*, *Pseudomonas aeruginosa*, *Klebsiella pneumoniae*, *Candida auris*, *Escherichia coli*, methicillin-resistant *Staphylococcus aureus*, *Acinetobacter baumannii*, *Enterobacter aerogenes*, and *Enterococcus faecalis* were diluted to a concentration of 1×10⁷ to 1×10⁸ CFU/mL to prepare inoculation suspensions. Then, 100 µL of the diluted inoculum suspension was added to 10 mL of 0.2 mM cobalt glutamate or cobalt aspartate solution (the aforementioned 0.1 M stock solution diluted 500-fold), resulting in a final concentration (i.e., initial concentration) of 1 × 10⁵ to 1 × 10⁶ CFU/mL. At 8, 24, and 48 hours after inoculation, 1.0 mL of sample was separately withdrawn to determine the viable cell count.

In the present embodiment, the bacteria used for the SGS antibacterial test were selected based on the World Health Organization Bacterial Priority Pathogens List (WHO BPPL). Priority was given to high-risk hospital-associated pathogens commonly referred to as "ESKAPE" in clinical settings, such as *Enterococcus*, *Staphylococcus aureus, Klebsiella pneumoniae, Acinetobacter baumannii, Pseudomonas aeruginosa*, and *Enterobacter*/*Klebsiella aerogenes*. "ESKAPE" is a term used in the field of clinical infections to refer to pathogens that easily evade immune responses or antibiotic treatment and frequently cause nosocomial infections or multidrug resistance. In this context, E stands for *Enterococcus faecium*, S stands for *Staphylococcus aureus*, K stands for *Klebsiella pneumoniae*, A stands for *Acinetobacter baumannii*, P stands for *Pseudomonas aeruginosa*, and E stands for *Enterobacter* (such as *Klebsiella aerogenes*)*.* These bacteria are considered representative of clinically high-risk drug-resistant pathogens.

### 1-2. Determination of Viable Bacterial Count

Prepare a 10-fold serial dilution of each 1.0 mL sample using soya casein digest lecithin polysorbate (SCDLP). Inoculate two tryptic soy agar (TSA) plates with the diluted samples and incubate at 30 to 35°C for 3 to 5 days. Calculate the number of viable pathogenic bacteria (CFU/mL) by determining the average number of colony-forming units (CFU) on the plates; the antibacterial activity R (%) is calculated as (initial bacterial count N0 - bacterial count after t hours Nt of treatment) / N0 × 100%.

### 1-3. Results of Cobalt Glutamate Against Various Pathogens

The antibacterial activity of cobalt glutamate against various pathogens is shown in Table 1 below.

**Table 1. Antibacterial Activity of Cobalt Glutamate Against Various Pathogenic Bacteria**

| Species | Initial Inoculum (CFU/mL) | Incubation Time (hr) | Sample Count at Incubation Time (CFU/mL) | Antimicrobial Activity R(%) |
|---|---|---|---|---|
| *Candida albicans* | 1.3 × 10⁵ | 8 | 1.3 × 10⁵ | < 1 |
| | | 24 | 4.4 × 10⁴ | 66.2 |
| | | 48 | 9.5 × 10³ | 92.7 |
| *Pseudomonas aeruginosa* | 3.6 × 10⁵ | 8 | 1.3 × 10³ | 99.6 |
| | | 24 | < 1 | > 99.9 |
| | | 48 | < 1 | > 99.9 |
| *Klebsiella pneumoniae* | 4.4 × 10⁵ | 8 | 3.1× 10⁵ | 29.5 |
| | | 24 | 8.5 × 10⁴ | 80.7 |
| | | 48 | 9.9 × 10³ | 97.8 |
| *Candida auris* | 6.6 × 10⁵ | 8 | 4.2 × 10⁵ | 36.4 |
| | | 24 | 2.6 × 10⁴ | 60.6 |
| | | 48 | 7.8 × 10⁴ | 88.2 |
| *Escherichia coli* | 7.0 × 10⁵ | 8 | 4.8 × 10⁵ | 31.4 |
| | | 24 | 5.9 × 10⁴ | 91.6 |
| | | 48 | 1.4 × 10⁴ | 98.0 |
| methicillin-resistant *Staphylococcus aureus* | 8.5 × 10⁵ | 8 | 7.7 × 10⁵ | 9.4 |
| | | 24 | 1.8 × 10⁴ | 97.9 |
| | | 48 | 9.0 × 10² | 99.9 |
| *Acinetobacter baumannii* | 3.0 × 10⁵ | 8 | 9.6 × 10⁴ | 68.0 |
| | | 24 | 2.8 × 10³ | 99.1 |
| | | 48 | 1.1 × 10² | > 99.9 |
| *Enterobacter aerogenes* | 6.2 × 10⁵ | 8 | 9.9 × 10⁴ | 84.0 |
| | | 24 | 1.5 × 10³ | 99.8 |
| | | 48 | 4.5 × 10¹ | > 99.9 |
| *Enterococcus faecalis* | 4.2 × 10⁵ | 8 | 1.6 × 10⁵ | 61.9 |
| | | 24 | 1.1 × 10⁵ | 73.8 |
| | | 48 | 5.8 × 10³ | 98.6 |

### 1-4. Results of Cobalt Aspartate Against Various Pathogens

The antibacterial activity of cobalt aspartate against various pathogens is shown in Table 2 below.

**Table 2. Antibacterial Activity of Cobalt Aspartate Against Various Pathogenic Bacteria**

| Species | Initial Inoculum (CFU/mL) | Incubation Time (hr) | Sample Count at Incubation Time (CFU/mL) | Antimicrobial Activity R(%) |
|---|---|---|---|---|
| *Candida albicans* | 2.4 × 10⁵ | 8 | 1.7 × 10⁵ | 29.2 |
| | | 24 | 1.2 × 10⁵ | 50.0 |
| | | 48 | 6.7 × 10³ | 97.2 |
| *Pseudomonas aeruginosa* | 6.0 × 10⁵ | 8 | 1.4 × 10⁴ | 97.7 |
| | | 24 | 2.1 × 10³ | 99.7 |
| | | 48 | 1.1 × 10³ | 99.8 |
| *Klebsiella pneumoniae* | 4.5 × 10⁵ | 8 | 2.0 × 10⁵ | 55.6 |
| | | 24 | 6.8 × 10⁴ | 84.9 |
| | | 48 | 4.3 × 10³ | 99.0 |
| *Candida auris* | 9.7 × 10⁵ | 8 | 7.9 × 10⁵ | 18.6 |
| | | 24 | 3.6 × 10⁵ | 62.9 |
| | | 48 | 2.6 × 10⁴ | 97.3 |
| *Escherichia coli* | 9.2 × 10⁵ | 8 | 4.7 × 10⁵ | 48.9 |
| | | 24 | 2.9 × 10⁵ | 68.5 |
| | | 48 | 1.7 × 10⁵ | 81.5 |
| methicillin-resistant *Staphylococcus aureus* | 9.7 × 10⁵ | 8 | 6.1 × 10⁴ | 93.7 |
| | | 24 | 5.6 × 10³ | 99.4 |
| | | 48 | 1.3 × 10² | > 99.9 |
| *Acinetobacter baumannii* | 3.0 × 10⁵ | 8 | 1.1 × 10⁵ | 63.3 |
| | | 24 | 4.0 × 10³ | 98.7 |
| | | 48 | 1.4 × 10² | > 99.9 |
| *Enterobacter aerogenes* | 6.2 × 10⁵ | 8 | 1.4 × 10⁵ | 77.4 |
| | | 24 | 1.3 × 10⁴ | 97.9 |
| | | 48 | 2.2 × 10³ | 99.6 |
| *Enterococcus faecalis* | 4.2 × 10⁵ | 8 | 5.8 × 10⁴ | 86.2 |
| | | 24 | 1.2 × 10⁴ | 97.1 |
| | | 48 | 5.5 × 10³ | 98.7 |

In the SGS antibacterial test, higher antibacterial activity indicates that the complex is more effective against pathogenic bacteria. The results in Tables 1 and 2 of Examples 1-3 and 1-4 above show that cobalt glutamate and cobalt aspartate exhibit good antibacteria effects against most pathogenic bacteria, including antibiotic-resistant pathogenic bacteria.

Cobalt glutamate and cobalt aspartate exhibited near-complete inhibition against most tested bacteria within 48 hours, with antibacterial rates ranging from approximately 98% to >99.9%; they also demonstrated inhibition rates of 92.7% and 88.2% against *Candida albicans* and *Candida auris*, respectively; among these, *Candida albicans* is a major pathogen responsible for invasive *Candida* infections and is closely associated with high in-hospital mortality rates and the burden of healthcare-associated bloodstream infections. *Candida auris* exhibits multidrug resistance and a tendency to cause hospital outbreaks.

In the present embodiment, cobalt glutamate and cobalt aspartate demonstrated near-complete inhibitory effects against the most clinically threatening drug-resistant pathogens, such as ESKAPE-associated strains, indicating their high potential as candidate drugs for "the treatment of multidrug-resistant bacterial infections".

### Example 2. Results of Antiviral Tests with Cobalt Glutamate

### 2-1. Preparation of Cells and Viral Strains

In the present embodiment, MDCK cells were cultured in liquid Dulbecco's Modified Eagle's Medium (DMEM) containing 10% fetal bovine serum (FBS) and incubated in a 37°C incubator with 5% carbon dioxide; After culturing, MDCK cells were dissociated using trypsin-EDTA, and the cell concentration was adjusted to 1.1 × 10⁵ cells/mL using 10% FBS-DMEM medium. Then, 200 µL was seeded into a 96-well plate and incubated in a 37°C incubator with 5% CO₂ for 18 to 24 hours and set aside.

Influenza A virus strains H1N1 and H3N2 were cultured in MDCK cells in 10% FBS-DMEM medium. When the cells reached 90% confluence, they were washed with phosphate-buffered saline (PBS). The cells were then seeded with a viral load corresponding to a multiplicity of infection (M.O.I) of 0.01 to allow for viral attachment and infection. 0% FBS-DMEM medium was added to replace the medium, using FBS-free DMEM to minimize serum interference with viral infection, replication, and assay results. The cells were then cultured in a 37°C incubator with 5% CO₂ for 48 hours; Once 50% of the cells exhibited cytopathic effects (CPE), all culture medium containing virus and CPE-infected cells was collected, centrifuged at 2000 rpm for 10 minutes, and the supernatant was collected.

### 2-2. Antiviral Effects of Cobalt Glutamate Against H1N1 and H3N2

The virus was diluted in DMEM. A total of 50 µL of the viral solution was mixed separately with 50 µL of cobalt glutamate diluted to 0.012 mM, 0.12 mM, 1.2 mM, 6 mM, and 30 mM, respectively. Then, 400 µL of DMEM was added to each mixture to bring the total volume of the virus-complex mixture to 500 µL for each concentration group, and the mixture was incubated at 37°C for 1 hour.

The cell supernatant was removed from the aforementioned 96-well plates, and the 500 µL complex-virus mixture described above was added. After incubation for 1 hour in a 37°C incubator with 5% CO₂, 3 mL of overlay medium was added, and the plates were cultured for 5 days in a 37°C incubator with 5% CO₂. Finally, formalin was added for fixation, the overlay medium was removed, and the cells were stained with crystal violet.

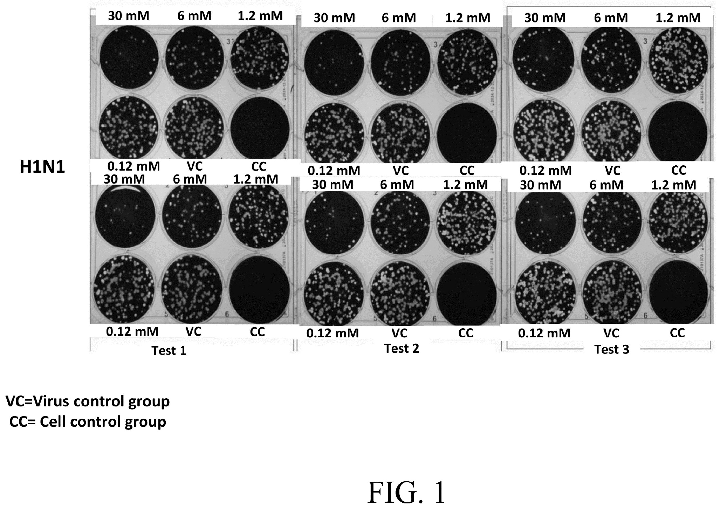

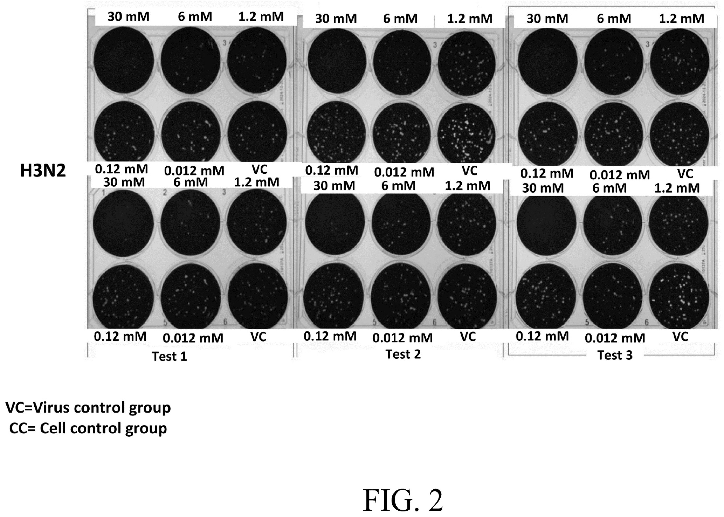

The results of cobalt glutamate inhibitory effects on H1N1 and H3N2 are shown in FIG. 1 and FIG. 2, and the quantitative results are presented in Tables 3 and 4 below.

**Table 3. Inhibitory effect of cobalt glutamate on H1N1**

| Concentration (mM) | Viral inhibition rate(%) | | |
|---|---|---|---|
| | H1N1 | | |
| | Test 1 | Test 2 | Test 3 |
| 0 | 0 | 0 | 0 |
| 0.12 | 2.7 | 5.02 | 0 |
| 1.2 | 10.13 | 0 | 0 |
| 6 | 54.05 | 53.51 | 44.72 |
| 30 | 85.71 | 84.95 | 75.47 |

**Table 4. Inhibitory effect of cobalt glutamate on H3N2**

| Concentration (mM) | Viral inhibition rate(%) | | |
|---|---|---|---|
| | H3N2 | | |
| | Test 1 | Test 2 | Test 3 |
| 0 | 0 | 0 | 0 |
| 0.012 | 0 | 0 | 0 |
| 0.12 | 0 | 2.37 | 20.98 |
| 1.2 | 10.84 | 21.3 | 33.57 |
| 6 | 59.04 | 66.86 | 61.54 |
| 30 | 86.75 | 86.4 | 85.31 |

The viral inhibition rate is a commonly used indicator in virology and the evaluation of antiviral drugs. It is calculated using the plaque assay as follows: Viral inhibition rate (%) = [1 - (P_treated - P_cell)/(P_virus - P_cell)] × 100; where P_cell represents the control group containing only cells, P_treated represents the experimental group treated with the test virus and the amino acid-metal complex, and P_virus represents the control group treated with the test virus alone.

Based on the results of the culture medium staining shown in FIGS. 1 and 2 and the quantitative data presented in Tables 3 and 4, cobalt glutamate exhibits good antiviral activity against both H1N1 and H3N2; specifically, at a concentration of 6 mM, it demonstrates significant inhibitory activity against both H1N1 and H3N2.

In summary, the amino acid-metal complexes of the present disclosure, namely cobalt glutamate and cobalt aspartate, exhibit significant antibacterial or antiviral effects. In contrast to the peptide-metal ions used in prior art, which achieve their inhibitory effects by disrupting bacterial cell membranes or viral envelopes but may simultaneously cause damage to non-target host cells. The single amino acid used in the present disclosure assists the chelated metal ions in the complex to enter bacterial cells to affect their metabolism, enter host cells to influence the viral environment within the body, or, through its G4 ligand properties, bind to the G4 structures of pathogens (including bacteria and fungi) or viruses, thereby affecting the replication, transcription, or gene expression of pathogens and viruses, and consequently achieving the effect of inhibiting their replication or proliferation. The mechanism of action of the present disclosure is milder and safer; the complexes formed by the chelation of amino acids with metal ions are also more stable; and the examples of the disclosure demonstrate great antibacterial or antiviral effects.

The present disclosure has been disclosed above in a preferred embodiment; however, those skilled in the art will understand that this embodiment is intended merely to illustrate the present disclosure and should not be construed as limiting the scope of the disclosure. It should be noted that any variations and substitutions equivalent to this embodiment should be considered to fall within the scope of the disclosure. Accordingly, the scope of protection of the present disclosure shall be defined by the claims.

## Claims

1. An amino acid-cobalt complex, wherein the amino acid is glutamic acid or aspartic acid.

2. The amino acid-cobalt complex according to claim 1, wherein if the amino acid is glutamic acid, its structure is represented by the following formula (I); if the amino acid is aspartic acid, its structure is represented by the following formula (II):

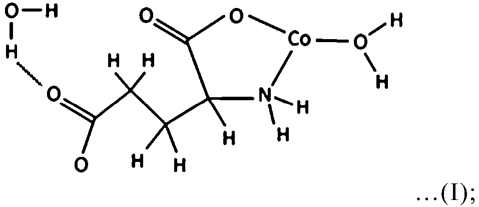

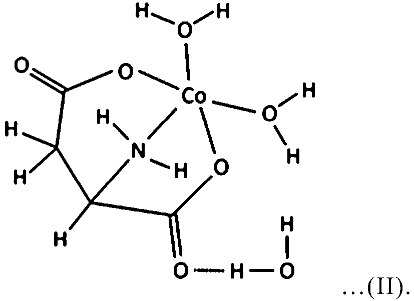

3. A use of the amino acid-cobalt complex according to claim 1, wherein the use is for the preparation of an antibacterial or antiviral composition against bacteria or viruses.

4. The use according to claim 4, wherein the bacteria is selected from the group consisting of *Candidatus Liberibacter* spp., *Xylella fastidiosa*, *Ralstonia solanacearum*, *Clavibacter sepedonicus* (synonyms: *Clavibacter michiganensis* subsp. *sepedonicus*), *Xanthomonas* spp., *Pectobacterium carotovorum*, *Dickeya* spp., *Erwinia amylovora*, *Pseudomonas syringae*, *Puccinia* spp., *Fusarium* spp., *Botrytis cinerea, Phytophthora infestans*, *Magnaporthe oryzae, Blumeria graminis*, *Erysiphe necator*, *Alternaria* spp., *Verticillium dahliae*, *Salmonella* spp., *Pasteurella multocida*, *Bordetella bronchiseptica*, *Clostridium perfringens*, *Staphylococcus aureus*/*Staphylococcus pseudintermedius*, *Mycobacterium bovis, Mycobacterium avium* subspecies *paratuberculosis*, *Brucella* spp., *Enterococcus faecium*, *Klebsiella pneumoniae*, *Acinetobacter baumannii*, *Pseudomonas aeruginosa*, *Klebsiella aerogenes* (synonyms: *Enterobacter aerogenes*), *Escherichia coli*, *Enterococcus faecalis*, *Haemophilus influenzae*, *Candida auris, Candida albicans*, *Cryptococcus neoformansi*, *Mycobacterium tuberculosis, Aspergillus fumigatus*, and *Neisseria gonorrhoeae.*

5. The use according to claim 4, wherein the virus is selected from the group consisting of avian influenza virus (AIV), foot-and-mouth disease virus (FMDV), porcine reproductive and respiratory syndrome virus (PRRSV), Newcastle disease virus (NDV), canine distemper virus (CDV), African swine fever virus (ASFV), influenza A/B/C/D virus, SARS-CoV-2, herpes simplex virus (HSV), human papilloma virus (HPV), respiratory syncytial virus (RSV), human immunodeficiency virus (HIV), hepatitis B virus (HBV), hepatitis C virus (HCV), dengue virus, and Zika virus.

6. The use according to claim 6, wherein the influenza A/B/C/D virus is H1N1 influenza A virus, H3N2 influenza A virus, or a combination thereof.

7. The use according to claim 4, wherein an antiviral concentration of the amino acid-cobalt complex is 6 to 30 mM.

8. The use according to claim 4, wherein an antibacterial concentration of the amino acid-cobalt complex is 100 mM or less.
